# EUROPEAN PATENT APPLICATION

(11) **EP 3 048 167 A1**
(43) Date of publication of application: **27.07.2016**
(21) Application number: 14846221.1
(22) Date of filing: 27.08.2014
(51) Int. Cl.: C12N 5/071, A01K 67/027, C12N 5/0775

(54) **METHOD FOR CREATING ENDOMETRIOTIC CELLS AND ENDOMETRIOSIS MODEL ANIMAL**

(30) Priority: 18.09.2013 JP 2013192596
(71) Applicant: Kyoto University, Kyoto-shi, Kyoto 606-8501 (JP); Astellas Pharma Inc., Chuo-ku Tokyo 103-8411 (JP)
(72) Inventor: KAJITANI, Takashi, Kyoto-shi Kyoto 606-8501 (JP)
(74) Representative: Brantsandpatents bvba
(86) International application number: PCT/JP2014/072499
(87) International publication number: WO 2015/041019

(57) **Abstract**

Provided is a method of producing a cell having properties of endometriosis (hereinafter referred to as "endometriotic-like cell") from a human mesenchymal stem cell. Also provided are a method of producing an animal model exhibiting human endometriosis, the method including transplanting the cell to an animal, and an animal model of endometriosis. The method of producing an endometriotic-like cell comprises a step of culturing a human mesenchymal stem cell induced to differentiate by allowing a differentiation inducing agent to act on the cell, through use of a low-carbon-source culture medium free of a differentiation inducing agent, in a culture vessel coated with extracellular matrix.

## Description

### Technical Field

The present invention relates to a cell having properties of endometriosis. The present invention also relates to a method of producing an animal model exhibiting human endometriosis, and to an animal model of endometriosis.

The present application claims priority from Japanese Patent Application No. 2013-192596, which is incorporated herein by reference.

### Background Art

Endometriosis is defined as a "disease in which a tissue having a form and a function identical or similar to those of endometrium ectopically grows and causes symptoms (Japan Society of Obstetrics and Gynecology, 1993)," and is a disease which is still unknown in many points including its development mechanism. Endometriosis is a disease which develops only in primates including humans, and it is difficult to construct a disease model of an endometriosis through use of generally used experimental animals, such as mice. In addition, an in vitro experiment using a tissue or a cell extirpated from a patient with endometriosis has problems in that a material is not regularly available and there is a large individual difference. Further, there is no established cell line having properties unique to endometriosis, and hence it has hitherto been difficult to continuously perform basic research and drug screening for elucidation of pathology.

It has been reported that cells or tissues of normal endometrium and endometriosis express specific differentiation markers, such as prolactin (PRL), insulin-like growth factor-binding protein 1 (IGFBP-1), and steroid hormone receptors [e.g., estrogen receptor 1 (ESR1), estrogen receptor 2 (ESR2), and progesterone receptor (PGR)] (Aghajanova et al., Endocrinology 151, 1341-1355, 2010; Tabanelli et al., The Journal of Steroid Biochemistry and Molecular Biology 42, 337-344, 1992; Tabibzadeh, Endocrine Reviews 12, 272-290 1991). In addition, it has been reported that the cell or tissue of endometriosis expresses or extracellularly secretes inflammatory factors, such as interleukin-1 receptor type 1 (IL-1Rt1), IL-6, IL-8, TNF receptor I (TNFR I), TNF receptor II (TNFR II), COX2, and MCP-1 (Bulun, New England Journal of Medicine 360, 268-279, 2009; Giudice and Kao, The Lancet 364, 1789-1799, 2004). Some attempts have hitherto been made to establish a cell line having such properties from an endometriotic tissue (Non Patent Literature 1: Bono et al., British Journal of Cancer 106, 1205-1213, 2012). However, the cell line cannot be said to faithfully reproduce a condition of endometriosis because of, for example, its deficiency in estrogen receptor-expressing ability.

A development mechanism of endometriosis is still unknown. However, it has been suggested that endometrium, which is considered as a development base, is embryologically an intermediate mesoderm-derived organ, and a mesenchymal stem cell in bone marrow, which is similarly derived from mesoderm, may differentiate to normal endometrium and an ectopic endometriotic tissue (Du and Taylor, Stem cells 25, 2082-2086, 2007; Taylor, JAMA: The Journal of the American Medical Association 292, 81-85, 2004). Meanwhile, it has been reported that a cell population similar to a mesenchymal stem cell is present in a normal endometrial tissue as well (Gargett et al., Biology of Reproduction 80, 1136-1145, 2009). In addition, it has been reported that the cell or tissue of endometriosis can synthesize an estrogen (estradiol: E2) from androgens of adrenal origin (dehydoepiandorosterone: DHEA and androstendione: A-dione), but cannot synthesize E2 from cholesterol de novo unlike adrenal glands and gonads (Bulun et al., Endocrine-Related cancer 6, 293-301, 1999).

There has been a previous report on research aimed at inducing differentiation from a mesenchymal stem cell to an endometrial cell (Non Patent Literature 2: Aghajanova et al., Biology of Reproduction 82, 1076-1087, 2010). However, this report is a report on research which is not aimed at inducing differentiation to a pathological endometriotic cell, but is aimed at inducing differentiation to a normal endometrial cell, and does not suggest induction of differentiation to an endometriotic cell.

### Citation List

### Non Patent Literature

[NPL 1] British Journal of Cancer 106, 1205-1213, 2012
[NPL 2] Biology of Reproduction 82, 1076-1087, 2010

### Summary of Invention

### Technical Problem

An object of the present invention is to provide a method of producing a cell having properties of endometriosis (hereinafter referred to as "endometriotic-like cell") from a human mesenchymal stem cell, a method of producing an animal model exhibiting human endometriosis, the method including transplanting the cell to an animal, and an animal model of endometriosis.

### Solution to Problem

The inventor of the present invention has focused attention on a method based on induction of differentiation from a mesenchymal stem cell instead of establishment from a patient-derived tissue. It has been reported that a cell or tissue of endometriosis expresses endometriotic differentiation markers and inflammatory factors, and the cell or tissue of endometriosis can synthesize an estrogen from androgens of adrenal origin, but cannot synthesize E2 from cholesterol de novo unlike adrenal glands and gonads. The inventor has aimed to develop an endometriotic-like cell capable of constructing a tissue similar to endometriosis through its transplantation to an immunodeficient mouse or the like while retaining such properties.

According to investigations made by the inventor of the present invention, it has been confirmed that a cell induced by the method of Non Patent Literature 2 (Aghajanova et al.) weakly expresses inflammatory factors and does not have the property of an estrogen synthesis ability from androgens of adrenal origin. In view of the foregoing, the inventor of the present invention has made extensive investigations. As a result, the inventor has advanced research with attention focused on induction of differentiation to a cell having properties of endometriosis, and has found that the intended object can be achieved by using a medium containing a carbon source at a low concentration, performing culture in a culture vessel coated with extracellular matrix, and further performing culture in the absence of a differentiation inducing agent after induction of differentiation. The present invention has been completed.

That is, the present invention includes the following.
1. A method of producing an endometriotic-like cell, the method comprising a step of culturing a human mesenchymal stem cell induced to differentiate by allowing a differentiation inducing agent to act on the cell, through use of a low-carbon-source culture medium free of a differentiation inducing agent, in a culture vessel coated with extracellular matrix.
2. A method of producing an endometriotic-like cell, the method comprising the following steps (1) to (3):
   (1) culturing a human mesenchymal stem cell with a low-carbon-source culture medium to proliferate the cell;
   (2) culturing the cell obtained in the step (1) with a low-carbon-source culture medium containing a differentiation inducing agent to induce the cell to differentiate; and
   (3) further culturing the cell obtained in the step (2) with a low-carbon-source culture medium free of a differentiation inducing agent,
   the culturing of each of the steps (1) to (3) being performed through use of a culture vessel coated with extracellular matrix.
3. A method of producing an endometriotic-like cell according to the above-mentioned item 1 or 2, in which the low-carbon-source proliferation culture medium contains about 500 mg/l to about 3,000 mg/l of glucose as a carbon source.
4. A method of producing an endometriotic-like cell according to any one of the above-mentioned items 1 to 3, in which the differentiation inducing agent is a cyclic AMP analog.
5. An endometriotic-like cell, which is produced by the production method of any one of the above-mentioned items 1 to 4.
6. A method of producing an animal model of endometriosis, the method comprising transplanting an endometriotic-like cell produced by the production method of any one of the above-mentioned items 1 to 4 to an immunodeficient animal.
7. An animal model of endometriosis, which is produced by the production method of the above-mentioned item 6.

### Advantageous Effects of Invention

In the endometriotic-like cell produced by the method of the present invention, the expression of various endometrial differentiation markers, and the expression of inflammatory factors found to be expressed at increased levels in an endometriotic tissue have been able to be confirmed. In addition, the endometriotic-like cell of the present invention also has an estrogen (E2) production ability from androgens of adrenal origin. Further, an animal model in which an endometriotic-like tumor lesion is confirmed has been able to be constructed by intraperitoneally injecting the endometriotic-like cell of the present invention to a female immunodeficient mouse.

### Brief Description of Drawings

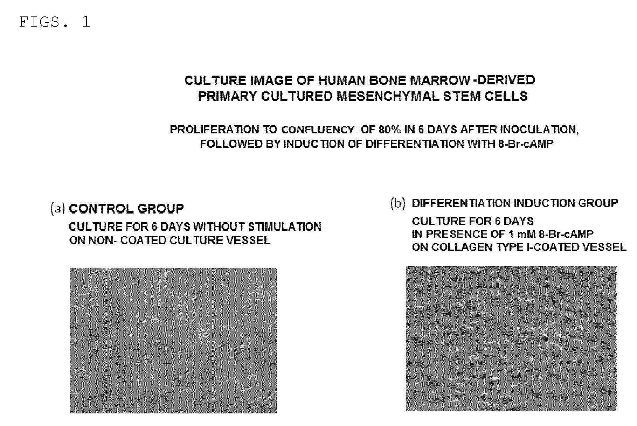

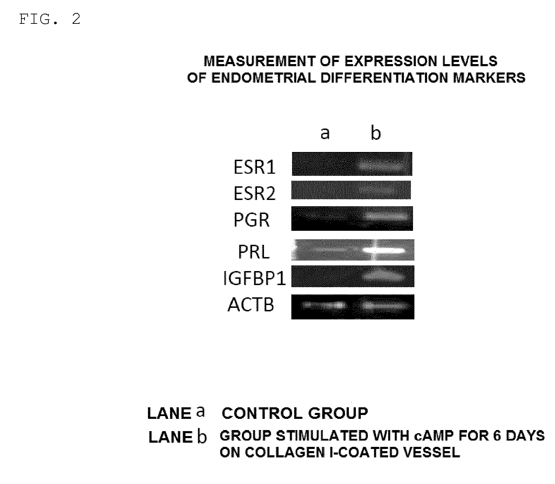

FIGS. 1 are photographs for showing cell morphology (culture image) after human bone marrow-derived primary cultured mesenchymal stem cells have been induced to differentiate by being cultured with a low-carbon-source culture medium supplemented with a differentiation inducing agent (8-Br-cAMP) through use of a culture vessel coated with extracellular matrix (Example 1, Experimental Example 1-1). FIG. 2 is a photograph for showing the results of confirmation of expression levels of various endometrial differentiation markers in the cells induced to differentiate (Example 1, Experimental Example 1-1)

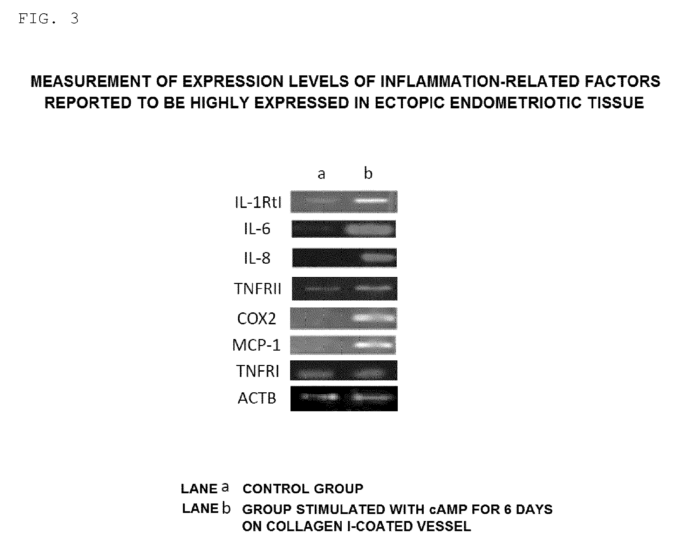

FIG. 3 is a photograph for showing the results of confirmation of expression levels of inflammatory factors found to be expressed at increased levels in an endometriotic tissue, in the cells induced to differentiate (Example 1, Experimental Example 1-1).

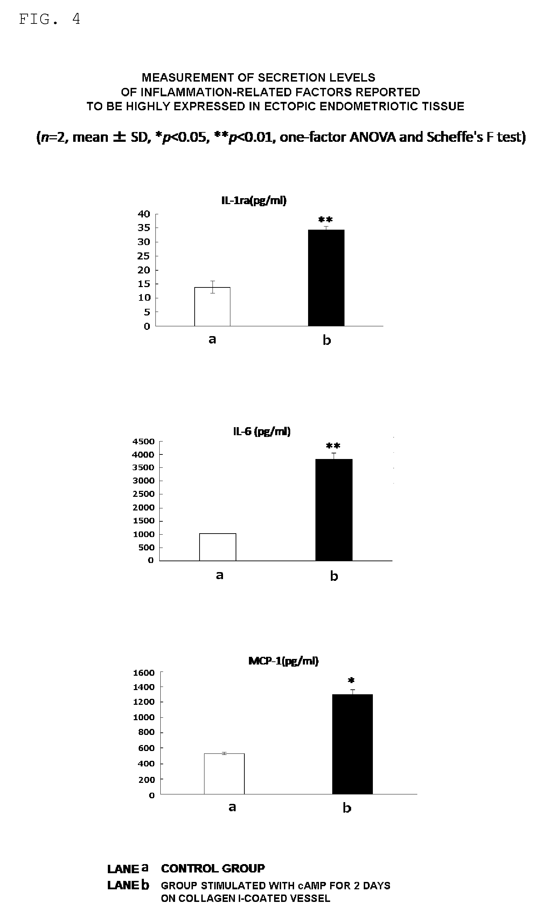

FIG. 4 is a graph for showing the results of confirmation of protein levels of inflammatory factors secreted into a cell culture supernatant in the cells induced to differentiate (Example 1, Experimental Example 1-1).

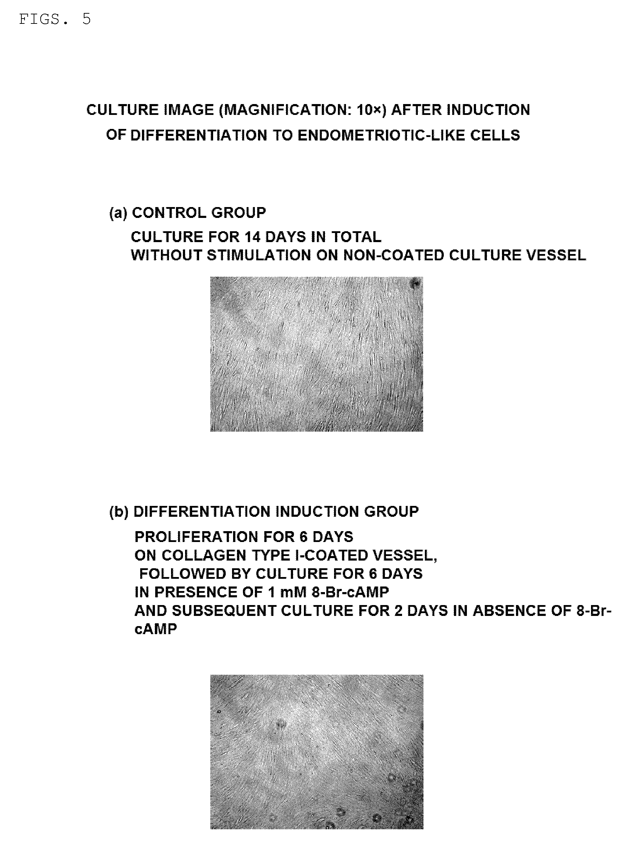

FIGS. 5 are photographs for showing cell morphology (culture image) after the cells have been induced to differentiate and then cultured with a low-carbon-source culture medium free of 8-Br-cAMP in exchange for the medium supplemented with 8-Br-cAMP (Example 1, Experimental Example 1-2).

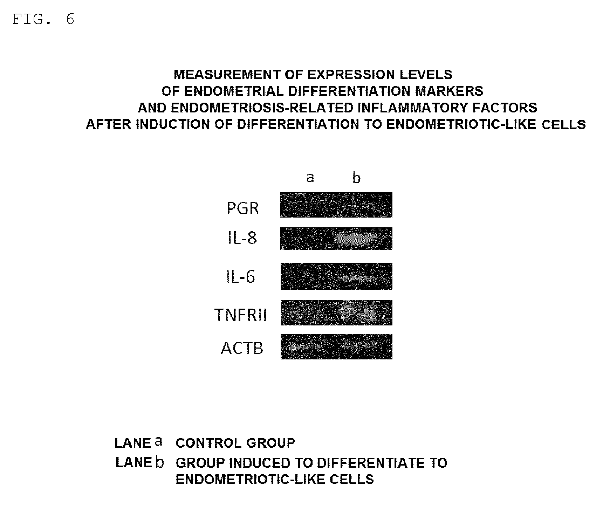

FIG. 6 is a photograph for showing the results of confirmation of the expression of endometrial differentiation markers and inflammatory factors after the cells have been induced to differentiate and then cultured with a low-carbon-source culture medium free of 8-Br-cAMP in exchange for the medium supplemented with 8-Br-cAMP (Example 1, Experimental Example 1-2).

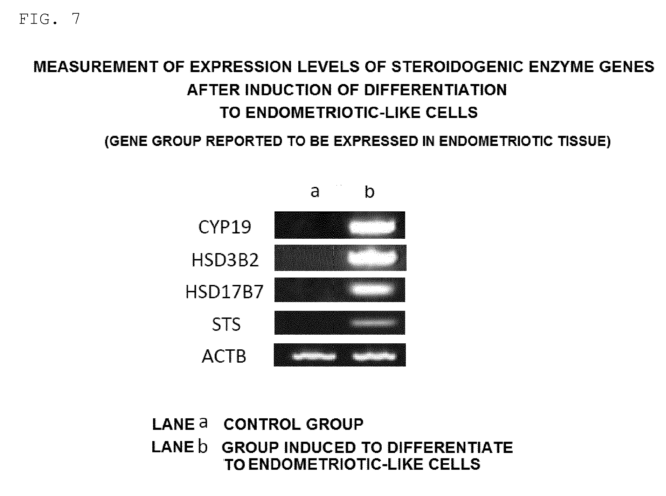

FIG. 7 is a photograph for showing the results of confirmation of the expression of steroidogenic enzyme genes CYP19, HSD3B2, HSD17B7, and STS, which are known to be highly expressed in an endometriotic tissue, in the cells induced to differentiate and then cultured with a low-carbon-source culture medium free of 8-Br-cAMP in exchange for the medium supplemented with 8-Br-cAMP (Example 1, Experimental Example 1-2).

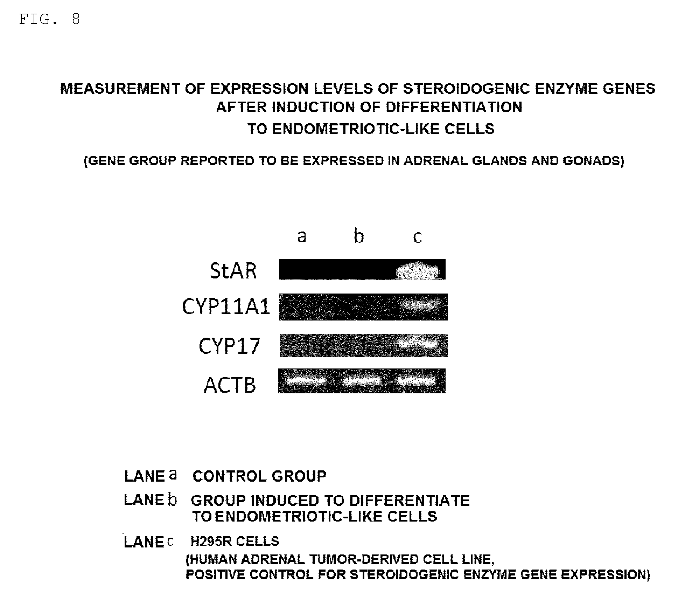

FIG. 8 is a photograph for showing the results of confirmation of the expression of StAR, CYP11A, and CYP17, which are known to be necessary for de novo synthesis of steroids in adrenal glands and gonads, in the cells induced to differentiate and then cultured with a low-carbon-source culture medium free of 8-Br-cAMP in exchange for the medium supplemented with 8-Br-cAMP (Example 1, Experimental Example 1-2).

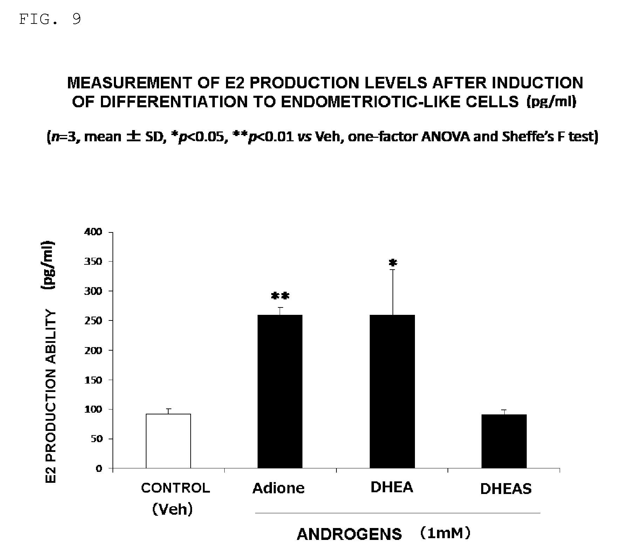

FIG. 9 is a graph for showing the results of confirmation of an estrogen(E2) production ability of the cells induced to differentiate and then cultured with a low-carbon-source culture medium free of 8-Br-cAMP in exchange for the medium supplemented with 8-Br-cAMP, in terms of the concentration of E2 in a culture supernatant (Example 1, Experimental Example 1-3).

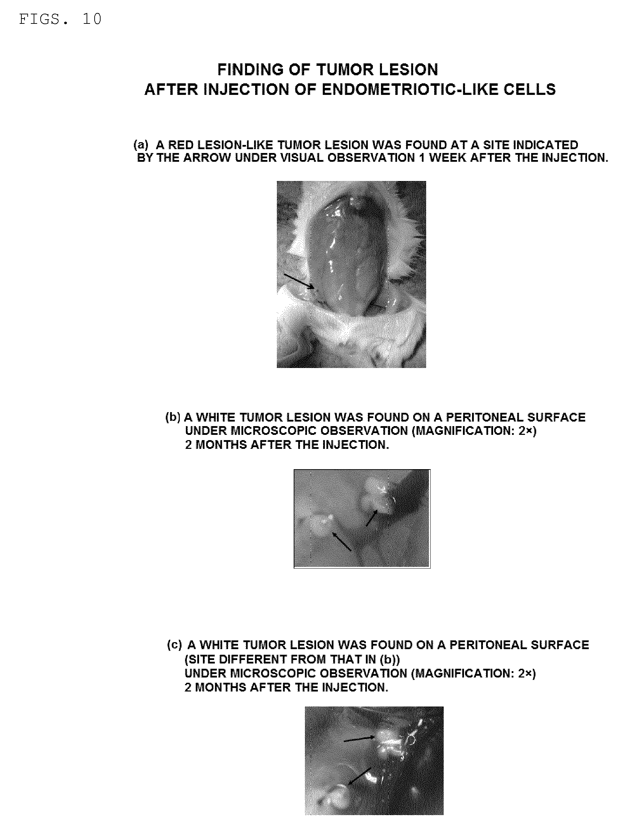

FIGS. 10 are photographs for showing an endometriotic-like tumor lesion formed in the peritoneal cavity (peritoneal surface) 1 week after injection (a) and 2 months after injection (b and c) in an animal model produced by intraperitoneally injecting endometriotic-like cells of the present invention to a female immunodeficient mouse (Example 2, Experimental Example 2-1).

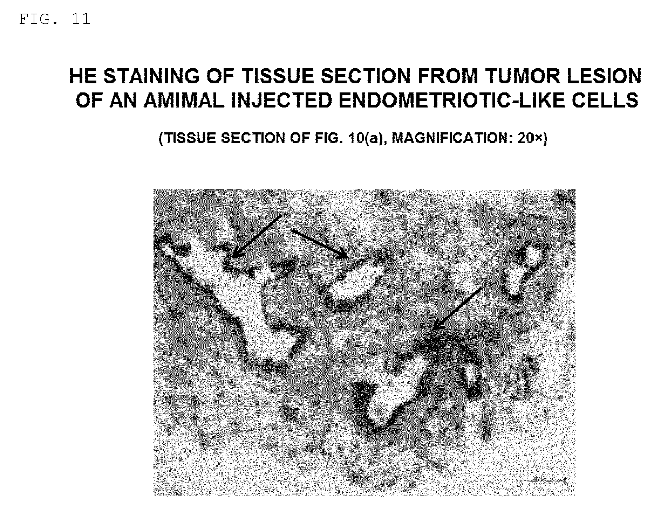

FIG. 11 is a photograph for showing the results of light microscopic observation of a tissue section produced from the confirmed tumor lesion and stained with hematoxylin-eosin (Example 2, Experimental Example 2-1).

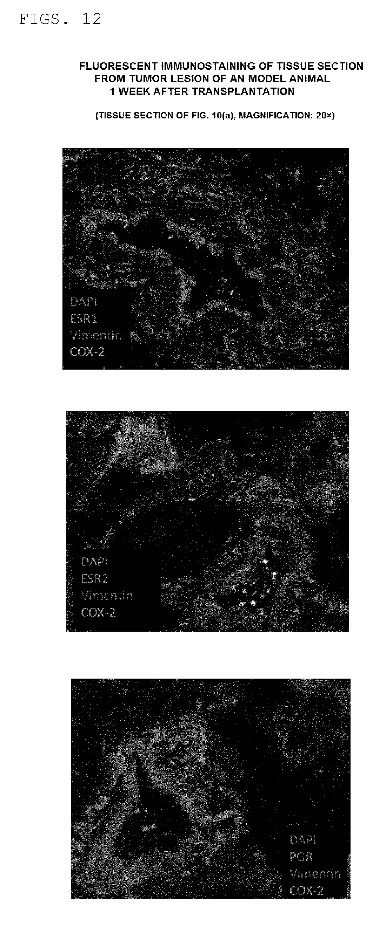

FIGS. 12 are photographs for showing the results of staining of a tissue section produced from the confirmed tumor lesion with endometrial markers and inflammatory markers found to be expressed in an endometriotic tissue as well (Example 2, Experimental Example 2-2).

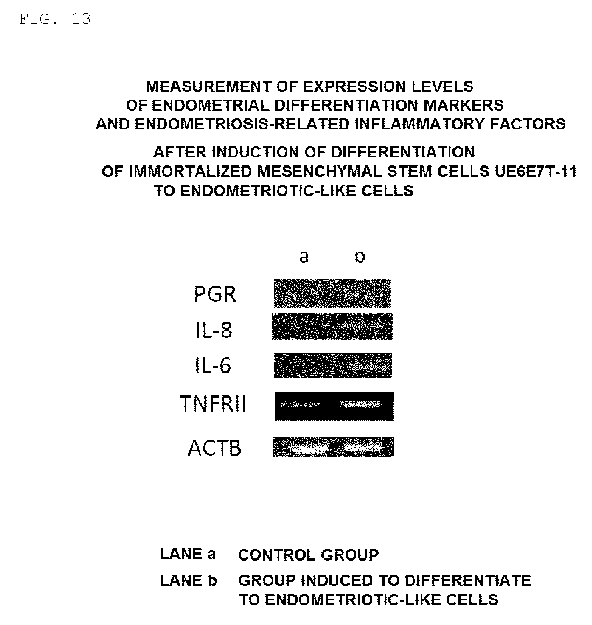

FIG. 13 is a photograph for showing the results of confirmation of the expression of endometriosis-related inflammatory factors (genes) in immortalized human mesenchymal stem cells UE6E7T-11 used in place of the human bone marrow-derived primary cultured mesenchymal stem cells when the cells are cultured in the same manner as in Example 1 (Example 3).

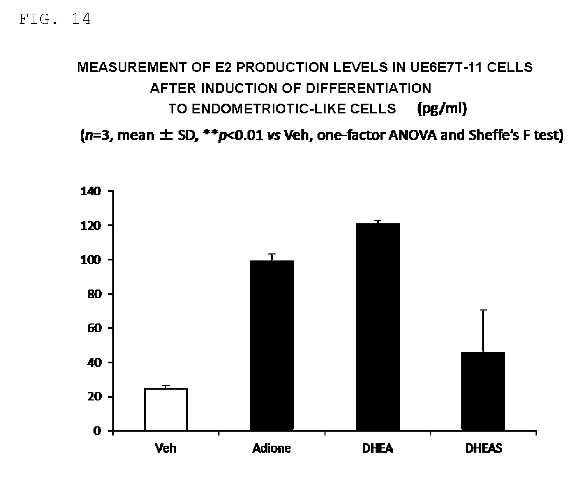

FIG. 14 is a graph for showing the results of analysis of an E2 production ability of immortalized human mesenchymal stem cells UE6E7T-11 used in place of the human bone marrow-derived primary cultured mesenchymal stem cells when the cells are cultured in the same manner as in Example 1 (Example 3).

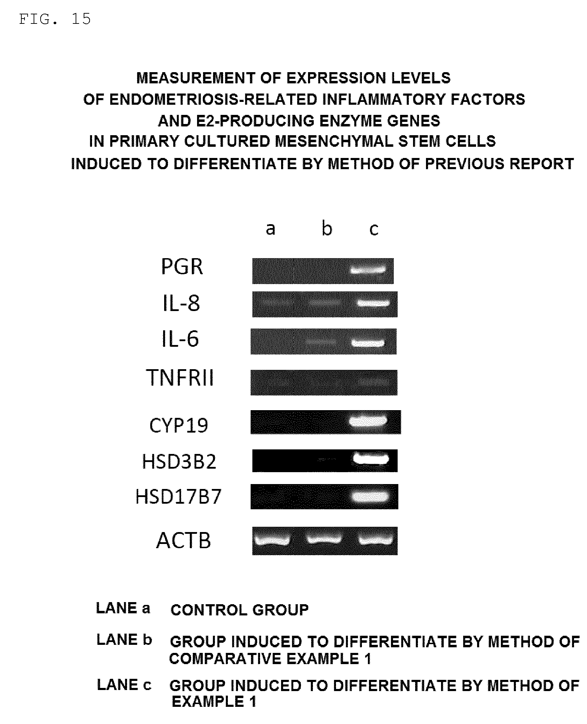

FIG. 15 is a photograph for showing the results of analysis of the expression of endometriosis-related inflammatory factors (genes) and E2-producing enzyme genes in cells obtained by culturing human bone marrow-derived primary cultured mesenchymal stem cells with a culture medium containing 4,000 mg/l of glucose through use of a culture vessel not coated with extracellular matrix (Comparative Example 1).

### Description of Embodiments

The present invention relates to a cell having properties of endometriosis, that is, an endometriotic-like cell. The present invention also relates to a method of producing an animal model exhibiting human endometriosis, and to an animal model of endometriosis. The endometriotic-like cell of the present invention is produced by a method comprising a step of culturing a human mesenchymal stem cell induced to differentiate by allowing a differentiation inducing agent to act on the cell, through use of a low-carbon-source culture medium free of a differentiation inducing agent, in a culture vessel coated with extracellular matrix.

More specifically, a method of producing an endometriotic-like cell of the present invention comprises the following steps (1) to (3):
(1) culturing a human mesenchymal stem cell with a low-carbon-source culture medium to proliferate the cell;
(2) culturing the cell obtained in the step (1) with a low-carbon-source culture medium containing a differentiation inducing agent to induce the cell to differentiate; and
(3) further culturing the cell obtained in the step (2) with a low-carbon-source culture medium free of a differentiation inducing agent,
the culturing of each of the steps (1) to (3) being performed through use of a culture vessel coated with extracellular matrix.

In the present invention, the "human mesenchymal stem cell" is a somatic stem cell derived from a human mesenchymal tissue, and any human mesenchymal tissue-derived somatic stem cell may be used. Examples thereof include bone marrow-derived, umbilical cord blood-derived, and adipose tissue-derived human mesenchymal stem cells, and commercially available products thereof (e.g., human bone marrow-derived primary cultured mesenchymal stem cells (Lonza)) may be used. Alternatively, human mesenchymal stem cells isolated from a human tissue through use of a method known in the art may be used. Established human mesenchymal stem cell lines may also be used. Examples thereof include: immortalized human bone marrow-derived mesenchymal stem cell lines, specifically UE6E7T-11, UE6E7T-12, and UE7T-13 (Takeda et al., The Journal of Gene Medicine 6, 833-845, 2004); and immortalized human umbilical cord blood-derived mesenchymal stem cell lines, specifically UCB-TERT-21 and UCB408E6E7TERT-33 (Terai et al., Molecular and Cellular Biology 16, 1491-1499, 2005) (these stem cell lines are available from Riken BioResource Center). In one embodiment, the human mesenchymal stem cell to be used in the present invention is a human bone marrow-derived mesenchymal stem cell.

In the present invention, the human mesenchymal stem cell is cultured in a culture vessel coated with "extracellular matrix." In the method of the present invention, any extracellular matrix for culture capable of serving as a scaffold for a tissue or a cell may be used as the "extracellular matrix." Examples thereof include components extracellularly secreted to enhance cell adhesion, i.e., collagen, proteoglycan, fibronectin, laminin, vitronectin, and hyaluronic acid which are obtained from a living body including a human, and artificially synthesized products of these proteins or polysaccharide (including a degradant and a fragmented product thereof), serum or plasma obtained from a living body including a human, and a product separated or purified therefrom. In addition, the extracellular matrix may be a commercially available extracellular matrix component (Matrigel^{™} matrix (manufactured by Becton, Dickinson and Company) or the like). More suitable examples thereof include type I collagen, proteoglycan, and fibronectin. The coating of the culture vessel with the exemplified extracellular matrix may be performed by a method known per se. For example, the culture vessel may be coated with type I collagen by adding 4 ml of a 50 mg/ml solution of type I collagen to a surface of a laboratory dish having a diameter of 100 mm. The culture vessel only needs to be a vessel to be generally used for cell culture.

In the present invention, the human mesenchymal stem cell is cultured with a "low-carbon-source culture medium." In the method of the present invention, the "low-carbon-source culture medium" refers to a culture medium containing a carbon source at a low concentration, and the carbon source to be used and its concentration may be appropriately selected by a person skilled in the art. An example of the carbon source contained in the low-carbon-source culture medium and its concentration is about 500 mg/l to about 3,000 mg/l, preferably about 500 mg/l to about 2,000 mg/l, more preferably about 500 mg/l to about 1,000 mg/l of glucose. As basal media for the low-carbon-source culture medium, there may be used, for example, media known in the art to be used for animal cell culture, such as commercially available low-glucose Dulbecco's Modified Eagle's Medium (DMEM, Vogt et al., Proceedings of the National Academy of Science of the United States of America 49, 171-179, 1963) (Nacalai Tesque, Inc.), αMEM medium, Ham's F12 medium, and RPMI 1640 medium. The medium may contain 5% (v/v) to 15% (v/v), preferably 8% (v/v) to 12% (v/v) of serum (e.g., foetal bovine serum (FBS)). In the case of using serum, it is preferred to remove low-molecular-weight compounds, such as a steroid hormone, in advance by a method known per se, for example, treatment with dextran-coated activated charcoal or the like. In addition, as necessary, the medium may contain, for example, an amino acid, L-glutamine, an antibiotic (e.g., penicillin, streptomycin, or amphotericin B), or Goods' buffer (4-(2-hydroxyethyl)-1-piperazineethanesulfonic acid (HEPES)) or the like for stabilizing a pH.

In the present invention, the "differentiation inducing agent" only needs to be a substance capable of inducing differentiation from human mesenchymal stem cells to cells expressing any endometrial markers (e.g., ESR1, ESR2, PGR, PRL, and IGFBP1) and endometriosis-related inflammatory factors (e.g., IL-1RtI, IL-1ra, IL-6, IL-8, TNFR I, TNFR II, COX2, and MCP-1). An example of such substance is a cyclic AMP (cAMP) analog. More specifically, any cAMP analog selected from the following analogs may be used: 8-bromoadenosine-3'-5'-cyclic monophosphate (8-Br-cAMP), dibutyryl-cAMP, and 8-(4-Chlorophenylthio)-2'-O-methyladenosine-3',5'-cyclic monophosphate sodium salt (8-CPT-2-Me-cAMP sodium salt). The concentration of the differentiation inducing agent to be used in the method of the present invention may be appropriately selected by a person skilled in the art depending on the differentiation inducing agent to be used. For example, in the case of using 8-Br-cAMP as the differentiation inducing agent, the concentration is from about 0.05 mM to about 5 mM, preferably from about 0.1 mM to about 2 mM.

In the culturing step of the method of the present invention, a culture temperature, a CO₂ concentration, and a culture time are appropriately selected by a person skilled in the art. The culture temperature is from about 30°C to about 40°C, preferably from about 36°C to about 38°C, the CO₂ concentration is from about 2% to about 5%, preferably about 5%, and the culture time is from about 2 days to about 8 days. Specific modes of the steps (1) to (3) of the method of the present invention are exemplified below.

### Step (1)

Human mesenchymal stem cells are added to a low-carbon-source culture medium and inoculated into a culture vessel coated with extracellular matrix. The number of the human mesenchymal stem cells to be inoculated may be appropriately selected, and for example, the cells are inoculated into the culture vessel at 2,500 cells/cm² to 5,000 cells/cm². It is preferred that the culture medium to be used in this step contain serum (e.g., FBS). In one embodiment, in this step, the human mesenchymal stem cells are cultured for from 6 days to 8 days under the conditions of from 36°C to 38°C in the presence of 5% CO₂. The culture medium is suitably exchanged every 2 days to 3 days. Through this step, the human mesenchymal stem cells are proliferated to a confluency of preferably 70% or more, more preferably from 70% to 80%.

### Step (2)

The human mesenchymal stem cells cultured in the step (1) are cultured with a low-carbon-source culture medium containing a differentiation inducing agent to induce the cells to differentiate. The differentiation inducing agent to be used and its concentration are as described above, and the cAMP analog is suitably added to the culture medium so that the final concentration became from 0.1 mM to 2 mM. In one embodiment, in this step, for the induction of differentiation, the cells are cultured for from 4 days to 8 days under the conditions of from 36°C to 38°C in the presence of 5% CO₂. The culture medium is suitably exchanged every 2 days to 3 days. It is preferred that the culture medium to be used in this step contain serum at almost the same concentration as that in the culture medium to be used in the step (1).

### Step (3)

After the induction of differentiation, the culture medium used in the step (2) is removed, and the cells obtained in the step (2) are further cultured with a low-carbon-source culture medium free of a differentiation inducing agent. In one embodiment, in this step, the cells are cultured for from 2 days to 4 days under the conditions of from 36°C to 38°C in the presence of 5% CO₂. It is preferred that the culture medium to be used in this step contain serum at almost the same concentration as that in the culture medium to be used in the step (1).

The endometriotic-like cells to be produced by the method of the present invention have the following properties 1) to 4): 1) expressing one or more differentiation markers unique to endometrium, such as ESR1, ESR2, PGR, PRL, and IGFBP-1; 2) expressing one or more inflammatory factors, such as IL-1Rt1, IL-1ra, IL-6, IL-8, TNFR I, TNFR II, COX2, and MCP-1; 3) expressing one or more steroidogenic enzyme genes, such as CYP19, HSD3B2, HSD17B2, and STS, which are known to be highly expressed in an endometriotic tissue, and not expressing StAR, CYP11A, and CYP17, which are known to be necessary for de novo synthesis of steroids in adrenal glands and gonads; and 4) producing an estrogen (E2) from androgens of adrenal origin. The present invention also encompasses "endometriotic-like cells" produced by the above-mentioned method, and the "endometriotic-like cells" of the present invention have the above-mentioned properties 1) to 4). The expression of various marker genes and proteins in the endometriotic-like cells may be confirmed by using a method known in the art. For example, a gene analysis method, such as RT-PCR or a hybridization method, is given as a method of confirming the expression of a gene, and an immunoassay, such as a Bio-Plex assay or a fluoroimmunoassay, is given as a method of confirming the expression of a protein.

An animal model of endometriosis which develops endometriotic-like lesions may be produced by transplanting the endometriotic-like cells obtained by the present invention to an immunodeficient animal, followed by breeding. Examples of the immunodeficient animal to be used include immunodeficient mice (e.g., NOD/Scid-IL-2R gamma null, CB17/Ic r -Prkdcscid/CrICrIj, and BALB/c Slc-nu/nu). For example, an animal model of endometriosis which develops endometriotic-like lesions at intraperitoneal sites may be produced by a method of producing an animal model of endometriosis using an immunodeficient mouse, the method including intraperitoneally transplanting the endometriotic-like cells (about 5×10⁵ cells/ml to about 2×10⁶ cells/ml) of the present invention suspended in about 0.1 ml to about 0.2 ml of phosphate-buffered saline (PBS) to a mouse, followed by breeding for from 1 week to 8 weeks.

### Examples

The present invention is more specifically described by way of Examples for further understanding of the present invention. However, it is apparent that the present invention is by no means limited to these Examples.

### (Example 1) Production of Endometriotic-like Cells

In this Example, endometriotic-like cells were produced from mesenchymal stem cells. Thus, procedures of the production are described in detail.

### Step (1) Culture of Mesenchymal Stem Cells

In this step, 2×10⁵ human bone marrow-derived primary cultured mesenchymal stem cells (Lonza, Cat. No. PT-2501) were added to 10 ml of a low-carbon-source proliferation culture medium (shown below), inoculated into a culture vessel having a diameter of 100 mm and having a surface coated with collagen type I (derived from rat tail, BD Biosciences, Cat. No. 354236), and cultured for 6 days to a confluency of from 70% to 80% at 37°C in the presence of 5% CO₂. The culture medium was exchanged every 2 days to 3 days.

In this Example and Experimental Examples shown below, and Examples shown below, the "low-carbon-source proliferation culture medium" refers to a culture medium prepared by supplementing low-glucose Dulbecco's Modified Eagle's Medium (containing 1,000 mg/l of glucose, phenol red-free, Nacalai Tesque, Inc., Cat. No. 08490-05), which serves as a basal medium, with L-glutamine at a final concentration of 2 mM, fetal bovine serum (FBS) at 10% (v/v), penicillin at a final concentration of 100 units/ml, streptomycin at a final concentration of 100 mg/ml, amphotericin B at a final concentration of 0.25 mg/ml, and 4-(2-hydroxyethyl)-1-piperazineethanesulfonic acid (HEPES) at a final concentration of 10 mM. FBS was treated with dextran-coated activated charcoal (Sigma, Cat. No. C6241) to remove low-molecular-weight compounds, such as a steroid hormone, in advance before use.

In this step, in a control group, the cells were cultured under the same conditions as those described above except for using a culture vessel not coated with extracellular matrix.

### Step (2) Induction of Differentiation of Mesenchymal Stem Cells

In this step, after the human bone marrow-derived primary cultured mesenchymal stem cells had been cultured to a confluency of from 70% to 80% in the step (1), the cells were induced to differentiate. A low-carbon-source proliferation culture medium supplemented with 8-Br-cAMP (Nacalai Tesque, Inc., Cat. No. 05450-86) so that the final concentration became 1 mM (sometimes referred to as "8-Br-cAMP(+) low-carbon-source proliferation culture medium") was prepared as a culture medium for the induction of differentiation. The cells (about 7×10⁵ cells to about 8×10⁵ cells) obtained in the step (1) were cultured for 6 days at 37°C in the presence of 5% CO₂ on the same culture vessel with only the medium being exchanged for 9 ml of a 8-Br-cAMP(+) low-carbon-source proliferation culture medium. The 8-Br-cAMP(+) low-carbon-source proliferation culture medium was exchanged every 2 days to 3 days.

In this step, in the control group, the cells obtained under the control conditions of the step (1) were cultured under the same conditions except for using a culture vessel not coated with extracellular matrix and using a low-carbon-source proliferation culture medium free of 8-Br-cAMP.

### Step (3) Production of Endometriotic-like Cells

In this step, after the induction of differentiation in the step (2), the medium was exchanged for a low-carbon-source proliferation culture medium free of 8-Br-cAMP, and the cells were further cultured. The cells (about 7×10⁵ cells to about 8×10⁵ cells) obtained in the step (2) were cultured for 2 days at 37°C in the presence of 5% CO₂ on the same culture vessel with only the medium being exchanged for 9 ml of a low-carbon-source proliferation culture medium. In this step, in the control group, the cells obtained under the control conditions of the step (2) were cultured under the same conditions except for using a culture vessel not coated with extracellular matrix.

### (Experimental Example 1-1) Analysis of Cells Induced to Differentiate in Step (2)

In this Experimental Example, the cells induced to differentiate in the step (2) were confirmed and analyzed for their cell morphology, expression of endometrial differentiation markers, and expression of endometriosis-related inflammatory factors (genes and proteins).

### (i) Cell Morphology

The morphology of the cells on day 6 after the start of the induction of differentiation in the step (2) was observed with a light microscope (magnification: 10×). As a result, as shown in FIG. 1(b), it was observed that, in the cells induced to differentiate with the medium supplemented with 8-Br-cAMP, the cells protruded to exhibit a round shape as compared to the control shown in FIG. 1(a), and had morphology similar to that of normal endometrial stromal cells decidualized in vitro.

### (ii) Expression of Endometrial Differentiation Markers

The cells on day 6 after the start of the induction of differentiation in the step (2) were confirmed for their expression levels of various endometrial differentiation markers, such as estrogen receptors (ESR1 and ESR2), progesterone receptor (PGR), prolactin (PRL), and insulin-like growth factor-binding protein 1 (IGFBP1). Total RNA was extracted from the cells (2×10⁵ cells) on day 6 after the start of the induction of differentiation through use of ReliaPrep RNA Cell Miniprep System (Promega, Cat. No. Z6012). After that, PrimeScript RT Master Mix (Takara Bio Inc., Cat. No. RR036B) was used to perform a reverse transcription reaction according to its manual. Thus, cDNA was synthesized. By using the cDNA as a template, Go Taq Green Master Mix (Promega, Cat. No. M7123) was used to perform a PCR reaction. The PCR product was subjected to agarose gel electrophoresis to measure expression levels of the endometrial differentiation markers. In that case, β-actin (ACTB) was used as an internal standard. The results are shown in FIG. 2 (lane a: control group, lane b: cAMP stimulation group). From the results, it was confirmed that the the expression of ESR1, ESR2, PGR, PRL, and IGFBP1 was increased.

### (iii) Expression of Endometriosis-related Inflammatory Factors (Genes)

The expression of inflammatory factors found to be expressed at increased levels in an endometriotic tissue was measured in the same manner as in the above-mentioned section (ii). As a result, as shown in FIG. 3, it was confirmed that the expression of interleukin-1 receptor type I (IL-1RtI), IL-6, IL-8, TNFR II, COX2, and MCP-1, which were known to be highly expressed in an ectopic endometriotic tissue, was increased by the induction of differentiation with 8-Br-cAMP. In this Example, little increase was found in the expression of TNFR I.

### (iv) Expression of Endometriosis-related Inflammatory Factors (Proteins)

The expression of inflammatory factors secreted into the cell culture supernatant on day 2 after the start of the differentiation induction in the step (2) at protein levels was measured by a Bio-Plex assay. More specifically, 50 µl of the supernatant was loaded into wells of Human Cytokine Assays 27-plex (Bio-Rad, Cat. No. M50-0KCAF0Y), and a cytokine contained in the supernatant was measured according to the manual. As a result, as shown in FIG. 4, it was confirmed that the levels of inflammatory factors IL-1ra (IL1 receptor antagonist), IL-6, and MCP-1 secreted into the culture medium were significantly increased.

### (Experimental Example 1-2) Analysis of Endometriotic-like Cells Produced in Step (3)

In this Experimental Example, the cells produced in the step (3), i.e., the cells induced to differentiate in the step (2) and then cultured for 2 days (total number of days for culture: 14 days) with a low-carbon-source proliferation culture medium free of 8-Br-cAMP through medium exchange again were analyzed.

### (i) Cell Morphology

Cell morphology was observed with a light microscope (magnification: 10×) in the same manner as in the above-mentioned section (i) of Experimental Example 1-1. The morphology of a control group (i.e., cells obtained after human mesenchymal stem cells have been cultured for 14 days with a low-carbon-source proliferation culture medium free of 8-Br-cAMP on a culture vessel not coated with extracellular matrix) is shown in FIG. 5(a), and the morphology of a differentiation induction group is shown in FIG. 5(b). As a result, as shown in FIG. 5(b), it was confirmed that, when the cells were cultured with the medium free of 8-Br-cAMP, the morphology of the cells returned to original fibroblast-like morphology similar to that of the control shown in FIG. 5(a).

### (ii) Expression of Endometrial differentiation Markers and Inflammatory Factors

The expression of endometrial differentiation markers and inflammatory factors was confirmed by the same techniques as in the above-mentioned sections (ii) and (iii) of Experimental Example 1-1. As a result, as shown in FIG. 6, when the cells were cultured with the medium free of 8-Br-cAMP, the expression of the endometrial differentiation marker PGR was reduced. On the other hand, it was confirmed that the expression of the inflammatory factors, such as IL-8, IL-6, and TNFR II, still remained high.

### (iii) Expression of Steroidogenic enzymes

The expression of steroidogenic enzyme genes CYP19, HSD3B2, HSD17B7, and STS, which were known to be highly expressed in an endometriotic tissue, was confirmed by the same techniques as in the above-mentioned sections (ii) and (iii) of Experimental Example 1-1. In addition, the expression of StAR, CYP11A, and CYP17, which were known to be necessary for de novo synthesis of steroids in adrenal glands and gonads, was similarly confirmed. As a result, as shown in FIG. 7, the expression of CYP19, HSD3B2, HSD17B7, and STS was increased. On the other hand, as shown in FIG. 8, no increase was found in the expression of StAR, CYP11A, and CYP17 (lane b).

### (Experimental Example 1-3) Estrogen (E2) Production Ability of Endometriotic-like Cells

In this Experimental Example, the cells produced in the step (3) were confirmed for their ability to produce estradiol (E2), which was an estrogen. The cells (1×10⁵ cells) produced in the step (3) were added to 1 ml of a low-carbon-source proliferation culture medium supplemented with bovine serum albumin (BSA) at 0.1% (v/v) in place of FBS, inoculated into a 12-well plate having a surface coated with collagen type I (derived from rat tail, BD Biosciences, Cat. No. 354236), and cultured for 24 hours at 37°C in the presence of 5% CO₂.

After that, A-dione (Tokyo Chemical Industry Co., Ltd.; androstenedione), DHEA (Tokyo Chemical Industry Co., Ltd.), or DHEA-sulfate (Tokyo Chemical Industry Co., Ltd.; DHEA-S, inactivated adrenal androgen), which was an androgen of adrenal origin, was added to the culture medium so that the final concentration became 1 mM, and the cells were further cultured for 48 hours. As a control (vehicle), the cells were cultured in the same manner as described above except that dimethylsulfoxide (DMSO), which was a solvent, was added in place of the androgen.

The concentration of E2 in the culture supernatant was measured by using Estradiol EIA kit (Cayman Chemical, Cat. No. 582251) according to the manual of the kit. As a result, as shown in FIG. 9, it was found that the cells produced in the step (3) produced E2 from A-dione and DHEA, but did not produce E2 from inactivated adrenal androgen (DHEA-S).

From the results of Example and Experimental Examples described above, it was confirmed that, when the human mesenchymal stem cells were induced to differentiate and then further cultured with a culture medium free of 8-Br-cAMP in exchange for the medium supplemented with 8-Br-cAMP, the expression of the endometrial differentiation marker PGR was reduced, but the expression of the endometriosis-related inflammatory factors (IL-8, IL-6, and TNFR II) still remained high. In addition, the expression of the steroidogenic enzyme genes CYP19, HSD3B2, HSD17B7, and STS, which were reported to be highly expressed in an endometriotic tissue, was increased, and an E2 production ability from androgens of adrenal origin (A-dione and DHEA) was confirmed, indicating that endometriotic-like cells were produced. On the other hand, no increase was found in the expression of StAR, CYP11A, and CYP17, which were essential for de novo synthesis of steroids, and hence it was confirmed that the cells produced in this case were not ones induced to differentiate to cells of adrenal or gonadal lineages.

### (Example 2) Production of Mouse Model of Endometriosis

100 µl of a PBS suspension (pH 7.4) of the endometriotic-like cells (1×10⁵ cells) produced in the step (3) of Example 1 was intraperitoneally injected to non-treated 8- to 14-week-old female immunodeficient mice (The Jackson Laboratory, US, NOD.Cg-Prkdc^{scid}Il2rg^{tm1wjl} /SzJ, abbreviated name: NSG). In this Example, as a control, cells obtained by culturing the human bone marrow-derived primary cultured mesenchymal stem cells (Lonza, Cat. No. PT-2501) used in Example 1 under the control conditions of the steps (1) to (3) of Example 1 in the stated order were intraperitoneally injected to the mice.

### ((Experimental Example 2-1) Confirmation of Endometriotic-like Tumor Lesion

When the mice were subjected to laparotomy under anesthesia 1 week after the injection of the cells to the mice, several endometriotic-like tumor lesions were confirmed in the peritoneal cavity (peritoneal surface). After a lapse of from 1 month to 2 months from the injection of the cells, both the number and size of the tumor lesions were increased. FIGS. 10 are photographs for showing the formed tumor lesions observed with the naked eye and under a stereoscopic microscope (magnification: 2x). On the other hand, such tumor lesion was not found in the control group.

The tumor lesion was excised, embedded in Tissue Tek O.C.T. Compound (Sakura Finetechnical), frozen, and then sliced to a thickness of 5 µm with a cryostat to produce a tissue section. The tissue section was subjected to hematoxylin-eosin staining according to an ordinary method and observed with a light microscope. As a result, as shown in FIG. 11, a glandular structure formed of epithelial-like cells surrounded by stromal cells, which was unique to endometriosis, was confirmed (arrow of FIG. 11).

### (Experimental Example 2-2) Confirmation of Endometrial markers and Inflammatory Markers

A tissue section produced in the same manner as in Experimental Example 2-1 was confirmed for its expression of ESR1, ESR2, and PGR, which were endometrial markers, and COX2, which was one of the inflammatory markers found in an endometriotic tissue as well. The tissue section was subjected to a reaction with each of rabbit anti-ESR1 antibody (Santa Cruz Biotechnology, Cat. No. sc-543), mouse anti-ESR2 antibody (abcam, Cat. No. ab288), mouse anti-PGR antibody (Dako, Cat. No. PgR636), and goat anti-COX2 antibody (Santa Cruz Biotechnology, Cat. No. sc-1746) serving as primary antibodies, and then subjected to a reaction with each of Alexa Fluor 488-labeled donkey anti-rabbit IgG antibody (Molecular Probe, Cat. No. A21206), Alexa Fluor 488-labeled donkey anti-mouse IgG antibody (Molecular Probe, Cat. No. A21202), and Alexa Fluor 647-labeled donkey anti-goat IgG antibody (Molecular Probe, Cat. No. A21447) serving as secondary antibodies. Finally, the tissue section was subjected to a reaction with Cy3-labeled mouse anti-vimentin antibody (Sigma, Cat. No. C9080) and mounted with a mounting agent ProLong Gold antifade reagent with DAPI (Molecular Probe, Cat. No. P36935) containing 4',6-diamidino-2-phenylindole (DAPI) as a nuclear staining agent. Thus, a fluorescent immunostaining section was produced. The section was observed by using a Zeiss confocal laser microscope LSM710 at a magnification of 20x. The results are shown in FIGS. 12.

The vimentin antibody used in this Experimental Example 2-2 is an antibody which does not react with a mouse antigen. Thus, it was confirmed that a red portion stained with the vimentin antibody was a stromal tissue derived from the injected human endometriotic-like cells. Epithelial-like cells having a glandular structure are present so as to be surrounded by the surrounding stromal tissue. In stromal and glandular epithelial-like tissues, cells positive for each of endometrial markers ESR1, ESR2, and PGR are present. In addition, cells positive for COX2, which is one of the inflammatory markers found in an endometriotic tissue as well are also present. From the results, it was confirmed that the tissue constructed from the cells transplanted into the mouse body was a tissue having properties similar to those of a human endometriotic tissue, suggesting that a mouse to which the cells were transplanted was able to be utilized as a mouse model of endometriosis.

### (Example 3)

Cell culture was performed in the same manner as in the steps (1), (2), and (3) of Example 1 except that, in the step (1) of Example 1, immortalized human mesenchymal stem cells UE6E7T-11 (available from Riken BioResource Center) were used in place of the human bone marrow-derived primary cultured mesenchymal stem cells (Lonza, Cat. No. PT-2501). The cells produced by such method were analyzed for their expression of endometriosis-related inflammatory factors (genes) and E2 production ability by the same techniques as in the confirmation in Experimental Example 1 of Example 1. The results are shown in FIG. 13 and FIG. 14. As shown in FIG. 13 and FIG. 14, the cells induced from UE6E7T-11 also highly expressed inflammatory markers and had an E2 production ability from androgens of adrenal origin.

From the results, it was confirmed that the cells produced from the immortalized human mesenchymal stem cells UE6E7T-11 were also endometriotic-like cells.

### (Comparative Example 1)

In this Comparative Example, human mesenchymal stem cells induced to differentiate by the method of the previous report (Non Patent Literature 2) were compared to the cells induced to differentiate by the method of Example 1. In this Comparative Example, in all the steps, a culture vessel not coated with extracellular matrix was used in place of the culture vessel coated with extracellular matrix used in Example 1. In addition, a culture medium in which the concentration of glucose was changed from 1,000 mg/l to 4,000 mg/l in the low-carbon-source proliferation culture medium used in Example 1 was used for each culture of this Comparative Example. Further, in conformity with the conditions of the previous report, the culture medium was supplemented with phenol red, and FBS not subjected to activated charcoal treatment was used. In addition, the concentration of L-glutamine in the culture medium was set to 4 mM, and HEPES was not added in conformity with the conditions of the previous report.

First, human bone marrow-derived primary cultured mesenchymal stem cells (Lonza, Cat. No. PT-2501) were proliferated and cultured in the same manner as in the step (1) of Example 1 except that the culture conditions were changed as described above.

Next, the cultured cells were cultured for 24 hours at 37°C in the presence of 5% CO₂ through use of a culture medium in which FBS was removed from the above-mentioned culture medium. After that, the cells were cultured and induced to differentiate in the same manner as in the step (2) of Example 1 through use of a culture medium prepared by changing the concentration of FBS in the above-mentioned culture medium from 10% (v/v) to 2% (v/v) and adding 8-Br-cAMP so that the final concentration became 1 mM.

The cells produced by such method were analyzed for their expression of endometriosis-related inflammatory factors (genes) and E2-producing enzyme genes by the same techniques as in the confirmation in Experimental Example 1 of Example 1. The results are shown in FIG. 15. In Comparative Example, only weak expression of inflammatory factors was found, and the expression of the E2-producing enzyme genes was not found. From the results, it was able to be confirmed that the endometriotic-like cells were not produced in the culture using the culture vessel not coated with extracellular matrix or the culture medium having a general carbon source concentration.

### Industrial Applicability

As described in detail above, it was able to be confirmed that the endometriotic-like cells produced by the method of the present invention expressed various endometrial differentiation markers, and also expressed inflammation-related factors, which were found to be expressed at increased levels in an endometriotic tissue. In addition, the endometriotic-like cells of the present invention also have an estrogen (E2) production ability. Further, an animal model in which an endometriotic-like tumor lesion was confirmed was able to be constructed by intraperitoneally injecting the endometriotic-like cells of the present invention to a female immunodeficient mouse.

The development mechanism of endometriosis is still unknown. In addition, although there is a report on research aimed at inducing differentiation from mesenchymal stem cells to endometrial cells, the research is not aimed at inducing differentiation to pathological endometriotic cells, but is aimed at inducing differentiation to normal endometrial cells. Thus, the report does not suggest induction of differentiation to endometriotic cells.

Accordingly, the construction of the endometriotic-like cells of the present invention and the construction of an animal model of endometriosis by transplanting such cells can contribute to the elucidation of the development mechanism of endometriosis, which has hitherto been unknown, can also contribute to the development of a preventive drug and a therapeutic drug, and by extension, can also contribute to a prevention method and a treatment method for endometriosis.

## Claims

1. A method of producing an endometriotic-like cell, the method comprising a step of culturing a human mesenchymal stem cell induced to differentiate by allowing a differentiation inducing agent to act on the cell, through use of a low-carbon-source culture medium free of a differentiation induction agent, in a culture vessel coated with extracellular matrix.

2. A method of producing an endometriotic-like cell, the method comprising the following steps (1) to (3):
(1) culturing a human mesenchymal stem cell with a low-carbon-source culture medium to proliferate the cell;
(2) culturing the cell obtained in the step (1) with a low-carbon-source culture medium containing a differentiation inducing agent to induce the cell to differentiate; and
(3) further culturing the cell obtained in the step (2) with a low-carbon-source culture medium free of a differentiation inducing agent,
the culturing of each of the steps (1) to (3) being performed through use of a culture vessel coated with extracellular matrix.

3. A method of producing an endometriotic-like cell according to claim 1 or 2, wherein the low-carbon-source proliferation culture medium contains about 500 mg/l to about 3,000 mg/l of glucose as a carbon source.

4. A method of producing an endometriotic-like cell according to any one of claims 1 to 3, wherein the differentiation inducing agent is a cyclic AMP analog.

5. An endometriotic-like cell, which is produced by the production method of any one of claims 1 to 4.

6. A method of producing an animal model of endometriosis, the method comprising transplanting an endometriotic-like cell produced by the production method of any one of claims 1 to 4 to an immunodeficient animal.

7. An animal model of endometriosis, which is produced by the production method of claim 6.
